# EUROPÄISCHE PATENTSCHRIFT

(11) **EP 2 366 377 B1**
(45) Veröffentlichungstag und Bekanntmachung des Hinweises auf die Patenterteilung: **04.12.2013**
(21) Anmeldenummer: 10002733.3
(22) Anmeldetag: 16.03.2010
(51) Int. Cl.: A61K 8/37, A61K 8/891, A61K 8/898, A61Q 5/02, A61Q 5/12

(54) **Konditionierungsmittel und konditionierende Shampoo-Zusammensetzung enthaltend Pentaerythrit-Ester**
Conditioner and conditioning shampoo comprising an ester of pentaerythritol
Composition de conditionnement et shampooing conditionnant comprenant un ester de pentaérythritol

(43) Veröffentlichungstag der Anmeldung: 21.09.2011
(73) Patentinhaber: Cognis IP Management GmbH, 40589 Düsseldorf (DE)
(72) Erfinder: Haake, Hans-Martin, 40699 Erkrath (DE); Prinz, Daniela, 41542 Dormagen (DE); Kano, Junko, Tokyo 106-6121 (JP); Doki, Kakushi, Tokyo 106-6121 (JP); Kim, Tae-Seong, Suji, Youngin, Gyeonggi (KR); Masaki, Koichi, Ibaraki 306-0213 (JP); Corneisen, Sybille, 40883 Ratingen (DE); Dierker, Markus, 40593 Düsseldorf (DE)

(56) Entgegenhaltungen:
- EP-A1- 1 792 604
- EP-A1- 2 077 139
- EP-A2- 1 216 685
- WO-A1-01/01949
- WO-A1-99/13839
- WO-A2-2008/012442
- JP-A- 2005 336 136
- DATABASE CA [Online] CHEMICAL ABSTRACTS SERVICE, COLUMBUS, OHIO, US; 7. Dezember 2006 (2006-12-07), YASUSHI, KO ET AL: "Hair-protecting compositions consisting of three agents" XP002589766 gefunden im STN Database accession no. 2006:1278391 & JP 2006 327954 A (ODA SEIYAKU K. K., JAPAN) 7. Dezember 2006 (2006-12-07)

## Beschreibung

### Gebiet der Erfindung

Die Erfindung befindet sich auf dem Gebiet der kosmetischen Konditionierungsmittel und der konditionierenden Shampoo-Zusammensetzungen, die Silikone und bestimmte Ester des Pentaerytbrits enhalten.

### Stand der Technik

Seit jeher werden Haarkonditionierungsmittel verwendet, um Struktur und Aussehen von Haaren zu verbessern. Durch Haarbehandlungen wie Färben oder Dauerwellen kommt es in den Haaren zu strukturellen Schädigungen. Das Haar wirkt spröde und matt, häufig spleißen die Haarenden auf. Insbesondere nach der Haarwäsche ist es fast unmöglich, das Haar zu kämmen.

Daher werden schon seit langem kosmetische Zubereitungen angeboten, die dem Abhilfe schaffen. Zum einen existieren seit langem am Markt 2-in-1 Shampoos, die als Shampoo mit integriertem Konditionierungsmittel wirken. Schon bei der Haarwäsche wird das Haar konditioniert, so dass sich das Haar nach dem Ausspülen direkt sehr gut kämmen läßt. Daneben gibt es auch noch Haarspülungen und Haarkuren, die separat nach der Haarwäsche angewendet werden.

Beliebte Inhaltsstoffe für alle diese Anwendungen sind Öle wie natürliche und ätherische Öle, Silikonöle, Fettalkohole und quaternäre Ammoniumverbindungen. Dabei hat sich gezeigt, dass es von wesentlicher Bedeutung ist, möglichst viel dieser Komponenten auf das Haar aufzubringen, um einen besonders guten Konditionierungseffekt zu erhalten.

JP-A-2005 336136 offenbart die Verwendung eines Pentaerythritolesters, insbesondere Pentaerythritol-tetra-2-ethylhexanoat zur Verbesserung der Abscheidung von aminomodifiziertem Silikon-Conditioner. JP-A-2005 336136 verwendet Pentaerythritoltetra-2-ethylhexanoat und keine konkrete Mischung der Ester des Pentaerythrits.

Aufgabe der vorliegenden Erfindung war es daher, Konditionierungsmittel bereit zu stellen, die durch Abscheidung hoher Mengen der eingesetzten Öle und insbesondere Silikonöle zu einer verbesserten Konditionierung der Haare führen.

### Beschreibung der Erfindung

Überraschenderweise wurde gefunden, die Abscheidung von Silikonölen auf Haare durch die Gegenwart von Fettsäureestern des Pentaerythrits erhöht werden kann. Ein erster Gegenstand der vorliegenden Erfindung ist daher eine kosmetische Zubereitung enthaltend
(a) Ester von Fettsäuren mit 4 bis 12 Kohlenstoffatomen mit Pentaerythrit mit einem Anteil an 5-35 Gew.-% Monoster, 20-50 Gew.-% Diester, 20-50 Gew.-% Triester und ggf. Tetraester und
(b) Silikone.

### Ester von Fettsäuren mit 4 bis 12 Kohlenstoffatomen und Pentaerythrit

Die Ester können eine einzige Art von Fettsäure-Acylgruppen aufweisen oder ein Gemisch verschiedener Fettsäure-Acylgruppen, die Fettsäuren können verzweigt oder unverzweigt und/oder gesättigt oder ungesättigt sein. Bevorzugt sind jedoch kürzere Alkylketten. Es ist davon auszugehen, dass hydrophilere Ester die Abscheidung der Silikone verstärken.

Bevorzugt werden daher für die Veresterung lineare oder verzweigte, gesättigte oder ungesättigte Fettsäuren mit Acylgruppen mit 4 bis 12 Kohlenstoffatomen eingesetzt. Besonders bevorzugt sind Acylgruppen mit 6 bis 10 Kohlenstoffatomen. Ganz besonders bevorzugt wird als Komponente (a) ein Ester von Pentaerythrit mit Caprylsäure eingesetzt. Zur Herstellung dieses besonders bevorzugten Esters werden 1 mol Pentaerythrit mit 2 mol Caprylsäure umgesetzt, wodurch ein Diester als Hauptkomponente anfällt. In einer bevorzugten Ausführungsform der Erfindung handelt es sich um Ester des Pentaerythrits mit einem Anteil an 5 - 35 Gew.-% Monoester, 20 - 50 Gew.-% Diester und 20 - 50 Gew.-% Triester, und ggf. Tetraester. Besonders bevorzugt ist ein Gehalt an 10 - 25 Gew.-% Monoester, 25 - 40 Gew.-% Diester und 30 - 40 Gew.-% Triester, und ggf. Tetraester und ganz besonders bevorzugt 15 - 25 Gew.% Monoester, 30 - 40 Gew.-% Diester, 25 - 35 Gew.-% Triester und 5 -11 Gew.% Tetraester. Gleichzeitig ist so sichergestellt, dass die Menge des nicht umgesetzten Pentaerythrits verschwindend gering bleibt (kleiner 0,5 Gew.%) und daher auch transparente und lichtdurchlässige Zubereitungen hergestellt werden können. Die vorzugsweise zu verwendenden Pentaerythritester sind flüssig, da sie so eine besonders gute Handhabung aufweisen und besser formulierbar sind. In den erfindungsgemäßen Zubereitungen werden die oben genannten Pentaerythritester in Mengen von 0,01 bis 5 Gew.%, bevorzugt von 0,1 bis 3 Gew.% und besonders bevorzugt von 1 bis 3 Gew.% eingesetzt.

### Silikone

Zur Konditionierung von Haaren werden in Shampoos, Haarspülungen, Haarkuren und dergleichen bevorzugt Silikone eingesetzt. Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können.

Im Rahmen der vorliegenden Erfindung hat es sich gezeigt, dass es besonders vorteilhaft ist, wenn als Komponente (b) Dimethicone oder Amodimethicone eingesetzt werden.

Üblicherweise werden die Silikone dabei in Mengen von 0,01 bis 5 Gew.% in den kosmetischen Zubereitungen eingesetzt und bevorzugt 1 bis 2 Gew.%.

### Kosmetische Zubereitungen

Die erfindungsgemäßen kosmetischen Zubereitungen können in Form von Haarshampoos, Haarlotionen, Haarspülungen, Haarkuren und dergleichen vorliegen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antioxidantien, Deodorantien, Antitranspirantien, Antischuppenmittel, Filmbildner, Quellmittel, Hydrotrope, Solubilisatoren, Konservierungsmittel, Parfümöle, Farbstoffe und dergleichen enthalten.

### Tenside

Shampoo-Zubereitungen enthalten in der Regel als eine der Hauptkomponenten Tenside zur Reinigung der Haare. Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate und -carboxylate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α -Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Neben den oben genannten Silikonen können die erfindungsgemäßen kosmetischen Zubereitungen jedoch auch noch weitere Ölkörper enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkoholen, substituierte Cyclohexane, lineare und verzweigte C₆-₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen und/oder aliphatische bzw. naphthenische Kohlenwasserstoffe, wie z.B. wie Squalan, Squalen oder Dialkylcyclohexane in Betracht.

### Emulgatoren

Weiterhin können die kosmetischen Zubereitungen der vorliegenden Erfindung, insbesondere wenn sie in Form von Cremes wie Haarkuren vorliegen, Emulgatoren enthalten. Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Zuckeralkoholen (z.B. Sorbit), Alkylglycosiden (z.B. Methylglucosid, Butylglucosid, Laurylglucosid) sowie Polyglycosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin,
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyaikylenglycole,
- Glycerincarbonat sowie
- Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht.

### Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Olsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsnuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Genzische. Ebenfalls geeignet sind Anlagerungsprodukte von 1 bis 30, vorzugsweise 5 bis 10 Mol Ethylenoxid an die genannten Sorbitanester.

### Polyglycerinester

Typische Beispiele für geeignete Polyglycerinester sind Polyglyceryl-2 Dipolyhydroxystearate (Dehymuls® PGPH), Polyglycerin-3-Diisostearate (Lamefrom® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glycerid, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpo-ly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium-und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat®) 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacotat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Oetylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β -Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, Ketoconazol, Elubiol, Selendisulfid, Schwefel kolloidal, Schwefelpolyehtylenglykolsorbitanmonooleat, Schwefelrizinolpolyehtoxylat, Sehwfelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglueoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Der Gesamtanteil der Hilfs- und Zusatzstoffe kann 1 bis 50, vorzugsweise 5 bis 40 Gew.% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

Ein weiterer Gegenstand der vorliegenden Erfindung ist die Verwendung von Estern von Fettsäuren mit 4 bis 12 Kohlenstoffatomen mit Pentaerythrit mit einem Anteil an 5-35 Gew.-% Monoester, 20-50 Gew.-% Diester, 20-50 Gew.-% Triester und ggf. Tetraester zur Erhöhung der Abscheidung von Silikonen aus konditionierenden kosmetischen Zubereitungen auf das Haar. Vorzugsweise werden Fettsäuren mit 6 bis 10 Kohlenstoffatomen im Acylrest verwendet.

### Beispiele

Um die Wirksamkeit der erfindungsgemäßen Zubereitungen zu belegen, wurden Haarsträhnen damit behandelt und die auf den Haaren abgeschiedene Menge von Silikon quantifiziert.

### Behandlung von Haarsträhnen

Fünf Haarsträhnen pro zu testender Formulierung wurden vorgereinigt, indem sie mit einer 6%ige Lösung von Texapon NSO (6% aktive Substanz, Sodium Laureth Sulfate, pH 6,5) gewaschen und anschließend gründlich ausgespült wurden. Die gereinigten Haarsträhnen wurden 20 min. mit Wasserstoffperoxid gebleicht (5% aktive Substanz, pH 9,4), gefolgt von gründlichem Ausspülen und einer einstündiger Trocknung (Luftstrom bei 55 °C). Alle beschriebenen Schritte wurden in einem automatischen System für die Präparierung von Haarproben durchgeführt.

### Behandlung mit Formulierungen 1- 4

Die fünf gebleichten Haarsträhnen pro Formulierung wurden für eine Minute in einer automatischen Spül- und Kämmvorrichtung befeuchtet. Danach wurden 0,125g Formulierung/1g Haar auf die feuchten Haarsträhnen aufgetragen. Nach drei Minuten Einwirkungszeit wurden die Haarsträhnen für eine Minute gespült in der automatischen Spül- und Kämmvorrichtung (bei 38 °C, 11/min für jede Haarsträhne). Die Haarsträhnen wurden mit warmer Luft (55°C)für eine Stunde getrocknet.

### Analytische Bestimmung der abgeschiedenen Silikonmenge

Die Menge von adsorbiertem Silikon am Haar wurde mit Hilfe von ICP-OES Analysen von Extrakten der Haarsträhnen bestimmt. Zu diesem Zweck wurde das Haar in Stücke geschnitten und das adsorbierte Silikon mit o-Xylen extrahiert. Die Extrakte wurden analysiert mit einem Vista MPX Radial (Varian Inc.) ICP Gerät. Als Standard diente ein zertifizierter Polydimethylsiloxan (PDMS) Kalibration Standard (Conostan®). Die Silikonkonzentration wurde aus den Silikonmengen multipliziert mit einem Faktor (2,64) errechnet, der aus dem reinen Standard bestimmt wurde. Dieser Faktor kann auch für modifizierte Silikone herangezogen werden, der der Derivatisierungsgrad relativ gering ist.

**Tabelle 1: Konditionierungsmittel enthaltend Silikone und Pentaerythritester und Menge des abgeschiedenen Silikons auf Haaren**

| **Inhaltsstoffe** | **1** | **2** | **3** | **4** |
|---|---|---|---|---|
| Dehyquart BT (Behentrimonium Chorid in Ethanol) | 1,3 | 1,3 | 1,3 | 1,3 |
| Dehyquart B (Steartrimonium Chlorid in Isopropanol) | 1,0 | 1,0 | 1,0 | 1,0 |
| Lanette O (Cetearyl Alcohol) | 4,0 | 4,0 | 4,0 | 4,0 |
| Pentaerythritdicaprylat | 1,0 | 3,0 | 1,0 | 3,0 |
| Cetiol OE (Dicaprylyl Ether) | 1,0 | 1,0 | 1,0 | 1,0 |
| Gluadin WLM (Hydolyzed Wheat Protein) | 1,0 | 1,0 | 1,0 | 1,0 |
| Aloveria (Aloe barbadensis leaf extract) | 1,0 | 1,0 | 1,0 | 1,0 |
| Dimethicone* | 3,0 | 3,0 | | |
| Amodimethicone** | | | 3,0 | 3,0 |
| Glycerin | 2,0 | 2,0 | 2,0 | 2,0 |
| p-Paraben | 0,15 | | | |
| m-Paraben | 0,15 | | | |
| Wasser | Ad 100 | | | |
| Silikon Abscheidung [µg/g Haa] | 304 | 289 | 320 | 305 |

| | | | | |
|---|---|---|---|---|
| * Dimethicone: SH200 200cs (Dow) ** Amodimethicone: KF-8004 (ShinEtsu) | | | | |

Alle Beispielformulierungen führen zu einer hohen Abscheidung von Silikon auf das Haar.

## Patentansprüche

1. Kosmetische Zubereitung enthaltend
(a) Ester von Fettsäuren mit 4 bis 12 Kohlenstoffatomen mit Pentaerythrit mit einem Anteil an 5 - 35 Gew.-% Monoester, 20 - 50 Gew.-% Diester, 20 - 50 Gew.-% Triester und ggf. Tetraester und
(b) Silikone.

2. Kosmetische Zubereitung gemäß Anspruch 1, **dadurch gekennzeichnet, dass** als Komponente (a) ein Ester von Pentaerythrit mit Caprylsäure eingesetzt wird.

3. Kosmetische Zubereitung gemäß einem der Ansprüche 1 oder 2, **dadurch gekennzeichnet, dass** als Komponente (b) Dimethicone oder Amodimethicone eingesetzt werden.

4. Kosmetische Zubereitung gemäß einem der Ansrpüche 1 bis 3, **dadurch gekennzeichnet, dass** die Zubereitung weiterhin quaternäre Ammoniumverbindungen enthält.

5. Kosmetische Zubereitung gemäß einem der Ansprüche 1 bis 4, **dadurch gekennzeichnet, dass** die Zubereitung weiterhin Wachse und Öle enthält.

6. Verwendung von Estern von Fettsäuren mit 4 bis 12 Kohlenstoffatomen mit Pentaerythrit mit einem Anteil an 5-35 Gew.-% Monoester, 20 - 50 Gew.-% Diester, 20 - 50 Gew.-% Triester und ggf. Tetraester zur Erhöhung der Abscheidung von Silikonen aus konditionierenden kosmetischen Zubereitungen auf das Haar.

## Claims

1. Cosmetic preparation comprising
(a) esters of fatty acids having 4 to 12 carbon atoms with pentaerythritol with a fraction of 5 - 35% by weight of monoester, 20 - 50% by weight of diester, 20 - 50% by weight of triester and optionally tetraester and
(b) silicones.

2. Cosmetic preparation according to Claim 1, **characterized in that** as component (a) an ester of pentaerythritol with caprylic acid is used.

3. Cosmetic preparation according to one of Claims 1 or 2, **characterized in that** as component (b) dimethicones or amodimethicones are used.

4. Cosmetic preparation according to one of Claims 1 to 3, **characterized in that** the preparation further comprises quaternary ammonium compounds.

5. Cosmetic preparation according to one of Claims 1 to 4, **characterized in that** the preparation further comprises waxes and oils.

6. Use of esters of fatty acids having 4 to 12 carbon atoms with pentaerythritol with a fraction of 5 - 35% by weight of monoester, 20 - 50% by weight of diester, 20 - 50% by weight of triester and optionally tetraester for increasing the deposition of silicones from conditioning cosmetic preparations on the hair.

## Revendications

1. Préparation cosmétique contenant
(a) des esters d'acides gras ayant de 4 à 12 atomes de carbone avec le pentaérythritol, avec une proportion de 5-35 % en poids de monoesters, 20-50 % en poids de diesters, 20-50 % en poids de triesters et éventuellement tétraesters et
(b) des silicones.

2. Préparation cosmétique selon la revendication 1, **caractérisée en ce qu'**on utilise comme composant (a) un ester de pentaérythritol avec l'acide caprylique.

3. Préparation cosmétique selon la revendication 1 ou 2, **caractérisée en ce qu'**on utilise comme composant (b) la diméthicone ou l'amodiméthicone.

4. Préparation cosmétique selon l'une quelconque des revendications 1 à 3, **caractérisée en ce que** la préparation contient en outre des composés d'ammonium quaternaire.

5. Préparation cosmétique selon l'une quelconque des revendications 1 à 4, **caractérisée en ce que** la préparation contient en outre des cires et des huiles.

6. Utilisation d'esters d'acides gras ayant de 4 à 12 atomes de carbone avec le pentaérythritol, avec une proportion de 5-35 % en poids de monoesters, 20-50 % en poids de diesters, 20-50 % en poids de triesters et éventuellement tétraesters, pour l'augmentation du dépôt de silicones sur le cheveu, à partir de préparations cosmétiques conditionnantes.
